(19) Europäisches Patentamt
European Patent Office
Office européen des brevets

(11) **EP 3 542 842 A1**

(12) **EUROPEAN PATENT APPLICATION**

(43) Date of publication:
**25.09.2019 Bulletin 2019/39**

(51) Int Cl.:
***A61M 5/142*** (2006.01)          ***H02P 8/00*** (2006.01)
***A61M 5/172*** (2006.01)

(21) Application number: **18305327.1**

(22) Date of filing: **23.03.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Fresenius Vial SAS**
**38590 Brézins (FR)**

(72) Inventor: **JHUBOO, Abdel-Nasser**
**38590 Saint Simeon de Bressieux (FR)**

(74) Representative: **Kusche, Robert**
**Fresenius Kabi Deutschland GmbH**
**Patent Department Medical Devices Division**
**Else-Kröner-Straße 1**
**61352 Bad Homburg (DE)**

(54) **INFUSION DEVICE COMPRISING A DRIVE MOTOR, IN PARTICULAR A BRUSHLESS DC MOTOR**

(57)    An infusion device (1) for administering a medical fluid towards a patient (3) comprises a pump mechanism (13) for delivering the medical fluid through an infusion line (2) towards the patient (3), a drive motor (14) for driving the pump mechanism (13), the drive motor (14) comprising an arrangement of phase windings (U, V, W) which are energizable to drive the pump mechanism (13), and a control device (15) for controlling the operation of the drive motor (14) by commutating the energization of the phase windings (U, V, W). Herein, the control device (15) is configured to commutate the energization of the phase windings (U, V, W) according to different modes of operation dependent on at least one of a characteristic of the medical fluid and an infusion setting for administering the medical fluid towards the patient (3). In this way an infusion device is provided which may be used in different environments, in particular a hospital environment as well as a homecare environment, taking requirements on an instantaneous infusion rate into account.

Fig. 3

EP 3 542 842 A1

## Description

The invention relates to an infusion device for administering a medical fluid towards a patient according to the preamble of claim 1.

An infusion device of this kind comprises a pumping mechanism for delivering the medical fluid through an infusion line towards the patient, a drive motor for driving the pump mechanism, the drive motor comprising an arrangement of phase windings which are energizable to drive the pump mechanism, and a control device for controlling the operation of the drive motor by commutating the energization of the phase windings.

Generally, infusion devices are used in different scenarios and environments. For example, within a hospital environment infusion devices are used at the bedside of a patient, for example in an intensive care unit, for delivering drugs to the patient. Within a hospital environment, infusion devices generally do not have to run on battery for an extended period of time, but due to their stationary use may be plugged into a wall socket for connection to a power supply. In contrast, in a homecare use an infusion device is typically run on battery for prolonged periods of time and should be portable such that a patient can carry the infusion device around without hindering the everyday life of the patient.

Different drugs generally have a different half-life. The biological half-life of a substance is generally understood as the time it takes for a substance, for example a drug, to lose half of its pharmacologic activity. Typically, this is due to the body's function to eliminate the substance from the body, a substance having a short half-life generally acting only over a rather short period of time within a patient's body, whereas a substance having a long half-life exhibits an activity within the patient's body over a prolonged period of time.

If infusing a drug at a low infusion rate, dependent on the drug's half-life the accuracy of the instantaneous infusion rate may have a significant impact. For a drug having a short half-life a variation in the instantaneous infusion rate may have a significant effect on the patient, such that an accurate instantaneous infusion rate is desired. In contrast, for a drug having a long half-life the instantaneous infusion rate even at low infusion rates does not play such a significant role.

For an infusion device within a hospital environment, commonly for example a DC stepper motor is used as drive motor, a DC stepper motor offering a beneficial accuracy of the instantaneous infusion rate even at low values of the infusion rate. However, a DC stepper motor generally has a rather poor efficiency, yielding a comparatively high power consumption.

In contrast, for ambulatory use for example in a homecare environment of a patient nowadays often a brushed DC electric motor is used, a brushed DC electric motor having a beneficial efficiency and low power consumption, hence allowing to run the infusion device on battery for an extended period of time, for example up to 24 hours or even longer. However, such a drive motor generally may not be able to offer an accurate instantaneous infusion rate at low values of the infusion rate.

It is an object of the instant invention to provide an infusion device which may be used in different environments, in particular a hospital environment as well as a homecare environment, taking requirements on an instantaneous infusion rate into account.

This object is achieved by means of an infusion device comprising the features of claim 1.

Accordingly, the control device is configured to commutate the energization of the phase windings according to different modes of operation dependent on at least one of a characteristic of the medical fluid and an infusion setting for administering the medical fluid towards the patient.

The pump mechanism is driven by the drive motor, which comprises phase windings. By energizing the phase windings a magnetic field is produced within the drive motor for rotating a rotor of the drive motor with respect to a stator in order to drive the pump mechanism. The energization of the phase windings herein is controlled by the control device, which controls the operation of the drive motor by commutating the current flowing through the phase windings such that a rotating magnetic field is produced within the drive motor.

Herein, dependent on for example a characteristic of the medical fluid, such as the half-life of the medical fluid, and an infusion setting for administering the medical fluid towards the patient, such as the infusion rate or a type of infusion therapy, the control device is configured to run the drive motor in different modes of operation. For this, the control device commutates the energization of the phase windings in a different manner, such that dependent on the mode of operation the drive motor exhibits a different motor behaviour.

By running the drive motor in different modes of operation in different scenarios, different motor characteristics may be obtained. In particular, for a drug having a short half-life the motor may be operated such that it provides for an accurate instantaneous infusion rate even at low values of the infusion rate. In contrast, for a drug having a long half-life (in which case an accurate instantaneous infusion rate is of lesser importance) the drive motor may be run such that it does not necessarily provide for an accurate instantaneous infusion rate, but provides for a beneficial efficiency and a low power consumption such that it may be run on battery for an extended period of time, for example in a homecare environment.

In addition, the type of infusion therapy may be taken into account. For example, for different infusion therapies an accurate instantaneous infusion rate may be of critical importance or not. For example one type of infusion therapy may be a pain management therapy. Another type of infusion therapy may be a nutritional feeding. Another type of infusion therapy may be the administration of antibiotics. Yet another type of infusion therapy may be a mean arterial pressure management therapy. Other types of infusion therapy may exist. For example within a type of infusion therapy such as a mean arterial pressure management therapy (within which a drug may be infused at a low flow rate) an accurate instantaneous infusion rate is of critical importance. For a pain management therapy or a nutritional feeding, in contrast, the accuracy of the instantaneous infusion rate may be of reduced importance, as an inaccurate instantaneous infusion rate within such therapies has little effect on the patient. Hence, by taking the type of infusion therapy into account an additional criteria for choosing the best mode of operation of the drive motor is considered.

In one embodiment, the drive motor comprises a stator carrying the phase windings and a rotor carrying an arrangement of permanent magnet poles and being rotatable with respect to the stator about a rotational axis. The drive motor in particular may be configured as a brushless direct current (DC) motor, the control device in particular serving to electronically commutating the phase windings of the stator such that by energizing the phase windings in a commutated fashion a rotating magnetic field is produced on the stator.

The use of a brushless direct current (DC) motor may offer the advantage of less wear and tear and hence a long operational life of the drive motor. In addition, such a drive motor may offer a beneficial operational behaviour even at high infusion rates, such as 1500 ml/h, as it may be required within a hospital environment for particular infusion therapies. A brushless direct current motor in particular may offer a reduced noise during operation, while at the same time being able to run at high speed and hence allowing to set large infusion rates.

For this, the control device for example comprises a switch arrangement suitable for electronically commutating the phase windings of the stator, the switch arrangement being constituted to energize the phase windings in an alternating fashion such that a desired magnetic field is produced on the stator. The switch arrangement may comprise a number of electronic switches, such as semiconductor switches, for electronically switching and energizing the phase windings.

In a first mode of operation the control device in particular may be configured to operate the drive motor in a step-controlled mode by continuously energizing the phase windings. Such a step-controlled mode offers a high accuracy of the instantaneous infusion rate even at low values of the infusion rate, but may have a reduced efficiency due to a comparatively large power consumption.

In the first mode of operation the control device may be configured to operate the drive motor by controlling the commutation of the energization of the phase windings in dependence on the rotational position and/or the rotation speed of the rotor by continuously energizing the phase windings to advance the rotors in controlled steps.

The drive motor is hence operated by using for example a feedback of a positional sensor for detecting the actual position and/or speed of the rotor, for example in the shape of an arrangement of Hall sensors for detecting the position and/or speed of the rotor. In such embodiments the commutation of the energization of the phase windings is sensor-controlled.

At low values of the infusion rate, as the position of the rotor is advanced in steps and hence continuously controlled, the speed of the rotor can be managed in a controlled way to optimize the instantaneous infusion rate in relationship with the pumping mechanism. However as the motor is permanently powered, the energy consumption is not optimized.

In a second mode of operation, in contrast, the drive motor may be run in a pulse mode by energizing the phase windings in periods of energization interrupted by periods of motor stoppage. The lengths of the periods of energization and the periods of motor stoppage herein are chosen such that on average an infusion rate equal to the desired infusion rate is obtained.

In the second mode of operation the instantaneous infusion rate in particular at low values of the infusion rate may not be quite as accurate. In turn, however, an improved efficiency due to a comparatively low power consumption is obtained, allowing to run the infusion device on battery for an extended period of time.

In one embodiment, during one period of energization the pump mechanism is driven by the drive motor to deliver a volume of the medical fluid towards the patient corresponding to a stroke volume of the pump mechanism. The stroke volume of the pump mechanism, in the context of a volumetric (peristaltic) infusion pump, may correspond to the volume of the medical fluid which is delivered towards the patient during for example one rotation of the pump mechanism comprising for example pump fingers for acting in a peristaltic fashion onto an infusion set. During one period of energization the pump mechanism hence is driven for one complete rotation of the pump mechanism such that a quantity of the medical fluid corresponding to the stroke volume of the pump mechanism is delivered to the patient. The control device then may be configured to set the period of motor stoppage in dependence on a desired infusion rate set by a user for administering the medical fluid towards the patient, such that on average over the periods of energization and the periods of motor stoppage the desired infusion rate is obtained.

It is also conceivable to deliver, during one period of energization, a volume of the medical fluid towards the patient corresponding to a portion of the stroke volume, for example 1/2, 1/3, 1/4 or 1/10 of the stroke volume. Hence, multiple periods of energization (pulses) are required to deliver one complete stroke volume. During one period of energization the drive motor, for example constituted as a brushless DC motor, is driven to deliver exactly the defined volume corresponding to a portion of the stroke volume. This allows to improve the accuracy of the instantaneous infusion rate.

By running the drive motor in different modes of operation the advantages of a step control similar to a stepper motor (offering a beneficial instantaneous infusion rate) are combined with the advantages of a brushless DC motor operated in a pulsed fashion (offering an improved efficiency due to low power consumption). Dependent on the drug to be administered to the patient, in particular the half-life of the drug, and dependent on the infusion rate which shall be obtained for administering the drug to the patient, and potentially furthermore dependent on a type of infusion therapy, a suitable mode of operation of the drive motor may be chosen, such that the infusion device may be beneficially used for example both in a hospital environment and in a homecare environment.

In one embodiment the control device is configured to compute, based on the mode of operation, a remaining time of use of a battery of the infusion device and to display the remaining time of use of the battery on a display of the infusion device. The control device hence computes over which remaining period of time the infusion device may be run on battery for the mode of operation which is currently set, that such that the user obtains a direct feedback about the remaining time the infusion device may be run on battery.

The idea underlying the invention set shall subsequently be described in more detail with respect to the embodiments shown in the figures. Herein:

Fig. 1    shows a schematic drawing of an infusion device for administering a medical fluid to a patient;

Fig. 2    shows a schematic, functional drawing of the infusion device;

Fig. 3    shows a schematic drawing of a drive motor and a control device of the infusion device;

Fig. 4    shows a graphical view of the energization of phase windings of a stator of the drive motor; and

Fig. 5    shows a graphical view of a pulsed energization of the phase windings in a pulsed mode of operation.

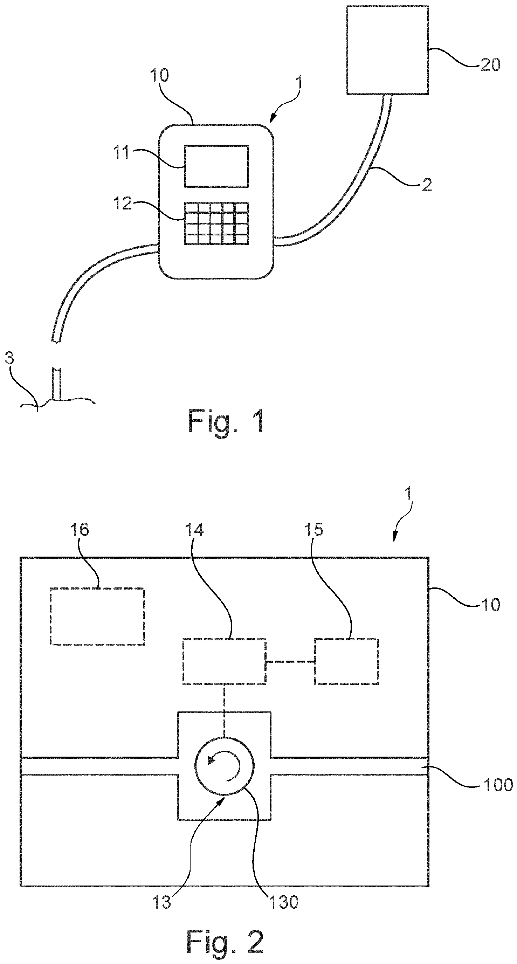

Fig. 1 shows a schematic view of an infusion device 1 configured to deliver a medical fluid such as a drug via an infusion line 2 from a container 20 towards a patient 3.

The infusion device 1 may for example be a volumetric (peristaltic) infusion pump having a housing 10 and a receptacle 100 formed therein, as schematically shown in Fig. 2, for receiving the infusion line 2 on the housing 10. The infusion device 1 comprises a display 11 and a keyboard 12 for entering pump settings for carrying out an infusion operation. The infusion device 1 furthermore comprises a pump mechanism 13 having for example a drive plate 130 rotatable to carry out a wobbling action to act on the infusion line 2 or to drive an arrangement of pump fingers to peristaltically pump a fluid through the infusion line 2 for advancing the medical fluid through the infusion line 2 towards the patient 3 for delivering the medical fluid towards the patient 3.

The infusion device 1 comprises a drive motor 14 controlled by a control device 15. The drive motor 14, for example by means of a suitable gearing, is in operative connection with the drive plate 130 such that by means of the drive motor 14 the pump mechanism 13 may be driven for delivering the medical fluid through the infusion line 2.

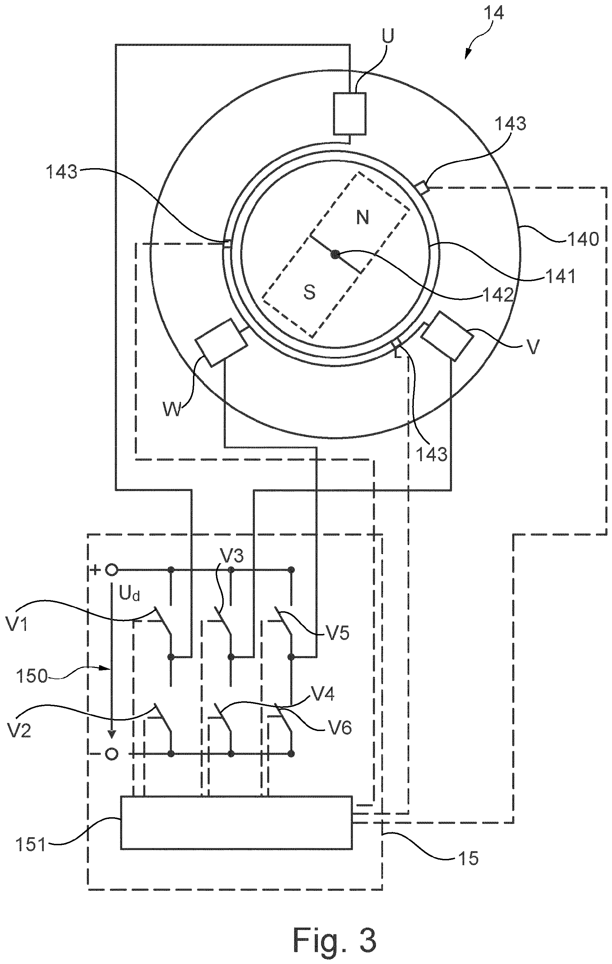

In the embodiment shown in Fig. 3, the drive motor 14 is formed by a brushless DC motor having a stator 140 and a rotor 141 being rotatable about a rotational axis 142 with respect to the stator 140. The stator 140 carries an arrangement of phase windings U, V, W forming three strands of windings which may be energized to produce a rotatable magnetic field on the stator 142 to interact with a magnetic field of permanent magnet poles N, S of a permanent magnet arrangement arranged on the rotor 141.

The stator 140, in one embodiment, carries an arrangement of positional sensors 143, for example in the shape of Hall sensors, for detecting the rotational position and/or speed of the rotor 141 with respect to the stator 140.

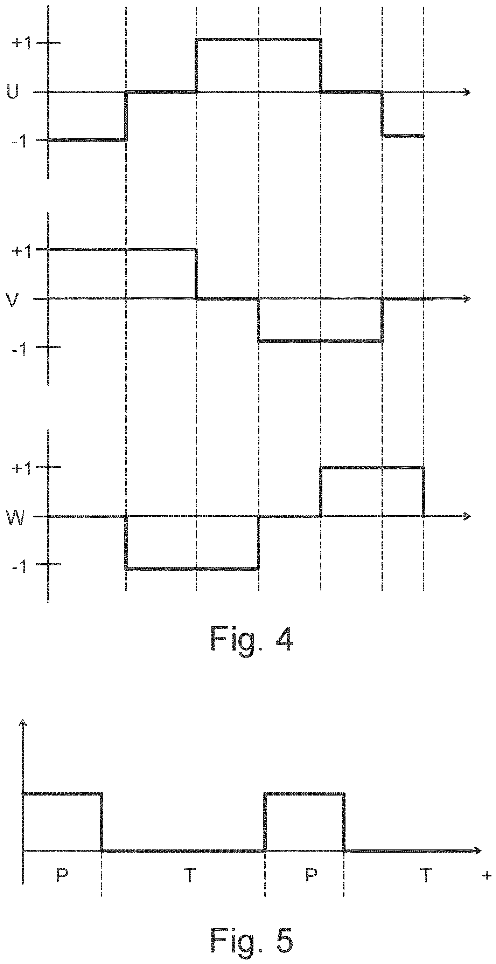

The control device 15 comprises a switch arrangement 150 for energizing the phase windings U, V, W of the drive motor 14 in an alternating fashion, as this graphically is shown in an example in Fig. 4 over time. The switch arrangement 150 comprises a number of (electronic) switches V1-V6 for sequentially connecting the phase windings U, V, W with a positive or a negative pole of a drive voltage Ud such that sequentially the positive and negative voltage Ud is switched onto the phase windings U, V, W, as is shown in Fig. 4, for producing a rotating magnetic field on the stator 140.

The control device 15 furthermore comprises an electronic controller 151 which controls the operation of the switches V1-V6 of the switch arrangement 150 and furthermore is in operative connection with the sensors 143 such that the controller 151 may take sensor signals of the sensors 143 into account for switching the switches V1-V6 for commutating the energization of the phase windings U, V, W of the stator 140.

The control device 15 is constituted to operate the drive motor 14 in different modes of operation.

In a first mode of operation the control device 15 controls the drive motor 14 to operate in a step-controlled fashion by commutating the energization of the phase windings U, V, W dependent of a positional feedback of the rotor 141, for example dependent of sensor signals of the sensors 143. By means of the switch arrangement 150 the phase windings U, V, W hence are continuously energized to produce a rotating magnetic field on the stator 140 to advance the rotor 141 in closely controlled positional steps.

For this, by means of the controller 151 the actual position and/or speed of the rotor 141, derived for example from sensor signals of the sensors 143 (for a sensor-controlled motor operation) or from a counter voltage arising in the phase windings U, V, W of the stator 140 (for a control of the motor operation in a sensor-less fashion), is taken into account such that the commutation of the energization of the phase windings U, V, W is controlled dependent on the actual position and/or speed of the rotor 141 with respect to the stator 140.

Such a step-controlled operation allows to obtain an accurate instantaneous infusion rate even at low values of the infusion rate (as low as 0.1 ml/h), at the expense of a reduced efficiency due to a rather high power consumption. In this way the infusion device 1 may be operated at very low infusion rates with a beneficial instantaneous accuracy, for example as low as 0.1 ml/h.

In a second mode of operation, in contrast, the control device 15 controls the drive motor 14 to run in a pulsed mode. Such an operation of the drive motor 14 offers a good efficiency due to a low power consumption, at the expense of a reduced accuracy in the instantaneous infusion rate in particular at low values of the infusion rate.

In the second mode of operation the drive motor 14 is controlled in a pulsed mode by energizing the drive motor 14 in periods of energization P interrupted by periods of motor stoppage T, as this is shown over time t in Fig. 5. During one period of energization herein the drive motor 14 may be driven to drive the pump mechanism 13 to deliver one stroke volume of medical fluid towards the patient 3, for example by driving the drive plate 130 to carry out one complete revolution. The volume of the medical fluid delivered through one period of energization P hence corresponds to the stroke volume. The control device 15 then is constituted to compute the period of motor stoppage T such that on average over the periods of energization P and the periods of motor stoppage T the desired infusion rate as is set by the user is obtained.

For example, if during one period of energization P a volume of the medical fluid corresponding to the stroke volume Vs is delivered to the patient, the period of motor stoppage T may be computed to have the length

$$T = Vs/R - P,$$

wherein R is the desired infusion rate.

In the pulsed mode of operation the instantaneous infusion rate is not very accurate, but only the overall, average infusion rate is accurate.

Alternatively, during one period of energization P a portion of the stroke volume Vs may be delivered to the patient 3. This may allow to improve the accuracy of the instantaneous infusion rate. For example, instead of delivering one complete stroke volume Vs at once, the stroke volume Vs may be split into N small volume portions Vsi. In this case the period of motor stoppage between two periods of energization Pi is computed to be

$$Ti = Vsi/R - Pi,$$

wherein one stroke volume Vs is delivered by delivering N small volume portions Vsi to the patient 3. The period of motor stoppage Ti is adjusted such that, on average, the desired infusion rate is obtained.

This may improve the accuracy of the instantaneous infusion rate. With a brushless DC motor even small volume portions Vsi can be delivered with a good volume accuracy.

Dependent on drug characteristics, in particular the half-life of a drug to be administered to a patient 3, an accurate instantaneous infusion rate is of great importance or not. In particular, for a drug having a short half-life an accurate instantaneous infusion rate is important. For a drug having a long half-life, in contrast, the instantaneous infusion rate does not necessarily need to be very accurate, as long as the overall, average infusion rate is accurate.

In addition, the type of infusion therapy may be considered to determine whether an accurate instantaneous infusion rate is required or not. For example, for a pain management therapy or for a nutritional feeding a high accuracy of the instantaneous infusion rate may not be required. In contrast, for example for a mean arterial pressure management therapy an accurate instantaneous infusion rate may be of critical importance.

For this reason the controller 15 is configured to operate the drive motor 14 in its different modes of operation dependent on a characteristic of the medical fluid to be administered to the patient 3, in particular the half-life of a drug to be administered to the patient 3, and in addition dependent on the infusion rate and/or the type of the infusion therapy for the administration.

For example, within an infusion therapy requiring an accurate instantaneous infusion rate and for a drug having a short half-life and a low infusion rate the control device 15 is configured to control the drive motor 14 to run in the step-controlled mode similar to a stepper motor by energizing the phase windings U, V, W continuously. If for example the infusion rate is below a first threshold and the half-life of the drug is below a second threshold the control device 15 may be configured to operate the drive motor 14 in the step-controlled mode.

The half-life of a drug as well as suitable thresholds to be used for operating the drive motor 14 may for example be stored in a drug library stored in the infusion device 1.

In contrast, for a drug having a long half-life or at high values of the infusion rate the control device 15 may be configured to operate the drive motor 14 in the pulsed mode. In this way the efficiency of the motor may be increased such that the infusion device 1 may be run on battery for an extended period of time.

In one embodiment, the control device 15 is configured to compute the remaining time of use of a battery 16 (see Fig. 2) of the infusion device 1. The remaining time of use of the battery 16 herein is computed by taking the mode of operation of the drive motor 14 into account. The remaining time of use indicates the time which is left to run the infusion device 1 on battery 16. The remaining time of use for example may be displayed on the display 11 of the infusion device 1.

Dependent on drug characteristics, in particular the half-life of a drug, and on infusion settings, in particular an infusion rate set by a user, the control device 15 hence chooses the best mode of operation for the drive motor 14 to obtain a high accuracy in the instantaneous infusion rate or an improved efficiency and low power consumption allowing to run the infusion device 1 on battery for an extended period of time. This allows to use the infusion device 1 both in a hospital environment (requiring for example a wide range of possible infusion rates between 0.1 ml/h and 1200 ml/h, but having reduced requirements on power efficiency) and in a homecare environment (in which infusion devices 1 are to be used over a smaller range of infusion rates for example in between 1 ml/h and 500 ml/h, but shall allow running on battery for an extended period of time, for example up to 48 hours).

The idea underlying the invention is not limited to the embodiments described above, but can be implemented in an entirely different fashion.

The infusion device may be a volumetric (peristaltic) infusion device or any other suitable infusion device.

The drive motor beneficially has the shape of a brushless DC motor offering different modes of commutating the energization of the phase windings. In principle, however, also other shapes of electric drive motors are possible.

**List of Reference Numerals**

| | |
|---|---|
| 1 | Infusion device |
| 10 | Housing |
| 100 | Receptacle |
| 11 | Display |
| 12 | Keyboard |
| 13 | Pump mechanism |
| 130 | Drive plate |
| 14 | Drive motor |
| 140 | Stator |
| 141 | Rotor |
| 142 | Rotational axis |
| 143 | Sensor device (Hall sensor) |
| 15 | Control device |
| 150 | Switch arrangement |
| 151 | Controller |
| 16 | Battery |
| 2 | Infusion line |
| 20 | Drug container |
| 3 | Patient |
| P | Period of energization |
| t | Time |
| T | Period of motor stoppage |
| Ud | Drive voltage |
| U, V, W | Phase windings |
| V1-V6 | Switch |

**Claims**

1. Infusion device (1) for administering a medical fluid towards a patient (3), comprising:

   - a pump mechanism (13) for delivering the medical fluid through an infusion line (2) towards the patient (3),
   - a drive motor (14) for driving the pump mechanism (13), the drive motor (14) comprising an arrangement of phase windings (U, V, W) which are energizable to drive the pump mechanism (13), and
   - a control device (15) for controlling the operation of the drive motor (14) by commutating the energization of the phase windings (U, V, W),

   **characterized in**
   **that** the control device (15) is configured to commutate the energization of the phase windings (U, V, W) according to different modes of operation dependent on at least one of a characteristic of the medical fluid and an infusion setting for administering the medical fluid towards the patient (3).

2. Infusion device (1) according to claim 1, **characterized in that** the at least one characteristic of the medical fluid relates to the half-life of the medical fluid.

3. Infusion device (1) according to claim 1 or 2, **characterized in that** the infusion setting relates to the infusion rate and/or a type of infusion therapy.

4. Infusion device (1) according to one of claims 1 to 3, **characterized in that** the drive motor (14) comprises a stator (140) carrying the phase windings (U, V, W) and a rotor (141) carrying an arrangement of permanent magnet poles (N, S) and being rotatable with respect to the stator (14) about a rotational axis (142).

5. Infusion device (1) according to claim 4, **characterized in that** the drive motor (14) is configured as a brushless direct-current motor.

6. Infusion device (1) according to claim 5, **characterized in that** the control device (15) comprises a switch arrangement (150) for electronically commutating the phase windings (U, V, W) of the stator (140).

7. Infusion device (1) according to one of claims 4 to 6, **characterized in that** the control device (15) is configured to operate the drive motor (14) in a first mode of operation in a step-controlled mode.

8. Infusion device (1) according to claim 7, **characterized in that** the control device (15) is configured to energize, in the first mode of operation, the phase windings (U, V, W) continuously.

9. Infusion device (1) according to one of claims 4 to 8, **characterized in that** the control device (15) is configured to energize the phase windings (U, V, W), in a second mode of operation, in a pulse mode by energizing the phase windings (U, V, W) in periods of energization (P) interrupted by periods of motor stoppage (T).

10. Infusion device (1) according to claim 9, **characterized in that** during one period of energization the pump mechanism (13) is driven to deliver a volume of the medical fluid towards the patient (3) corresponding to a stroke volume or a portion of the stroke volume of the pump mechanism (13).

11. Infusion device (1) according to claim 9 or 10, **characterized in that** the control device (15) is configured to set the period of motor stoppage (T) in dependence on a set infusion rate for administering the medical fluid towards the patient (3).

12. Infusion device (1) according to one of claims 4 to 11, **characterized in that** the control device (15) is configured to operate the drive motor (14) by controlling the commutation of the energization of the phase windings (U, V, W) in dependence on the rotational position and/or the rotational speed of the rotor (141).

13. Infusion device (1) according to one of the preceding claims, **characterized in that** the control device (15) is configured to compute, based on the mode of operation, a remaining time of use of a battery (16) of the infusion device (1) and to display the remaining time of use of the battery (16) on a display (11) of the infusion device (11).

Fig. 1

Fig. 2

Fig. 3

Fig. 4

Fig. 5

Europäisches
Patentamt

European
Patent Office

Office européen
des brevets

# EUROPEAN SEARCH REPORT

Application Number

EP 18 30 5327

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 849 240 B2 (MEDTRONIC MINIMED INC [US]) 26 December 2017 (2017-12-26) * column 12, lines 10-38; figure 5 * ----- | 1-13 | INV. A61M5/142 H02P8/00 A61M5/172 |
| X | EP 1 178 599 A1 (ALARIS MEDICAL SYST INC [US]) 6 February 2002 (2002-02-06) * paragraphs [0014] - [0017] * * paragraphs [0027] - [0033]; figures 1-9 * * paragraphs [0003], [0004] * ----- | 1-13 | |
| X | US 2016/339171 A1 (TROCK ADAM S [US] ET AL) 24 November 2016 (2016-11-24) * paragraph [0036]; figure 4 * ----- | 1-13 | |
| X | US 4 898 579 A (GROSHONG LEROY E [US] ET AL) 6 February 1990 (1990-02-06) * figures 1-3,14 * ----- | 1-13 | |
| X | US 2012/078217 A1 (SMITH ROGER E [US] ET AL) 29 March 2012 (2012-03-29) * paragraphs [0500], [0501] * ----- | 1-13 | TECHNICAL FIELDS SEARCHED (IPC) A61M H02P |

The present search report has been drawn up for all claims

| Place of search | Date of completion of the search | Examiner |
|---|---|---|
| Munich | 23 October 2018 | Krassow, Heiko |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 18 30 5327

This annex lists the patent family members relating to the patent documents cited in the above-mentioned European search report.
The members are as contained in the European Patent Office EDP file on
The European Patent Office is in no way liable for these particulars which are merely given for the purpose of information.

23-10-2018

| Patent document cited in search report | | | Publication date | Patent family member(s) | | | Publication date |
|---|---|---|---|---|---|---|---|
| US 9849240 | B2 | | 26-12-2017 | US | 2015165119 | A1 | 18-06-2015 |
| | | | | US | 2018071456 | A1 | 15-03-2018 |
| EP 1178599 | A1 | | 06-02-2002 | AT | 215282 | T | 15-04-2002 |
| | | | | AT | 364926 | T | 15-07-2007 |
| | | | | CA | 2231644 | A1 | 20-03-1997 |
| | | | | DE | 862811 | T1 | 06-05-1999 |
| | | | | DE | 69620219 | D1 | 02-05-2002 |
| | | | | DE | 69620219 | T2 | 18-07-2002 |
| | | | | DE | 69637134 | T2 | 14-02-2008 |
| | | | | DK | 1178599 | T3 | 15-10-2007 |
| | | | | EP | 0862811 | A1 | 09-09-1998 |
| | | | | EP | 1178599 | A1 | 06-02-2002 |
| | | | | ES | 2120390 | T1 | 01-11-1998 |
| | | | | ES | 2288494 | T3 | 16-01-2008 |
| | | | | HK | 1015976 | A1 | 19-07-2002 |
| | | | | HK | 1044078 | A1 | 11-01-2008 |
| | | | | JP | H11512596 | A | 26-10-1999 |
| | | | | PT | 1178599 | E | 18-09-2007 |
| | | | | US | 6016044 | A | 18-01-2000 |
| | | | | US | 6211642 | B1 | 03-04-2001 |
| | | | | US | 2002008490 | A1 | 24-01-2002 |
| | | | | WO | 9710642 | A1 | 20-03-1997 |
| US 2016339171 | A1 | | 24-11-2016 | US | 2015025499 | A1 | 22-01-2015 |
| | | | | US | 2016339171 | A1 | 24-11-2016 |
| US 4898579 | A | | 06-02-1990 | NONE | | | |
| US 2012078217 | A1 | | 29-03-2012 | NONE | | | |

EPO FORM P0459

For more details about this annex : see Official Journal of the European Patent Office, No. 12/82